# EUROPEAN PATENT APPLICATION

(11) **EP 4 534 667 A1**
(43) Date of publication of application: **09.04.2025**
(21) Application number: 23815756.4
(22) Date of filing: 16.05.2023
(51) Int. Cl.: C12N 15/11, C12N 1/15, C12N 1/19, C12N 1/21, C12N 5/10, C12N 15/63, C12P 1/04

(54) **MODIFIED PROMOTER, EXPRESSION VECTOR, MICROORGANISM, PRODUCTION METHOD FOR SUBSTANCE, MODIFIED CYANOBACTERIA, AND MANUFACTURING METHOD FOR MODIFIED PROMOTER**

(30) Priority: 31.05.2022 JP 2022088371
(71) Applicant: Panasonic Intellectual Property Management Co., Ltd., Kadoma-shi, Osaka 571-0057 (JP)
(72) Inventor: WAKABAYASHI, Masaya, Kadoma-shi, Osaka 571-0057 (JP); KUSAMA, Shoko, Kadoma-shi, Osaka 571-0057 (JP); KOJIMA, Seiji, Kadoma-shi, Osaka 571-0057 (JP)
(74) Representative: Grünecker Patent- und Rechtsanwälte PartG mbB
(86) International application number: PCT/JP2023/018190
(87) International publication number: WO 2023/233996

(57) **Abstract**

The present disclosure provides a modified promoter with which the amount of expression of a protein-coding gene can be controlled with flexibility. The modified promoter according to the present disclosure is a modified promoter including a base sequence (SEQ ID NO: 1) located upstream of a start codon of a slr1841 gene, the base sequence consisting of 348 bases, or a base sequence having 80% or more homology with the base sequence represented by SEQ ID NO: 1, the base sequence being modified by at least one selected from the group consisting of (i) replacement of one or more bases included in the base sequence with another base, (ii) deletion of one or more bases included in the base sequence, and (iii) insertion of one or more bases into the base sequence.

## Description

### Technical Field

The present disclosure relates to a modified promoter, an expression vector including the modified promoter, a microorganism including the expression vector, a method for producing a substance in which the microorganism is used, a modified cyanobacterium including the modified promoter introduced in the genome, and a method for preparing a modified promoter.

### Background Art

Promoters capable of increasing the amount of expression of a gene coding a substance (e.g., a protein) that is to be produced by microorganisms have been developed in order to increase the productivity of substances using microorganisms.

For example, PTL 1 discloses a knowledge that modifying the base sequence of a promoter of the alkaline cellulase gene of bacillus subtilis enhanced the activity of the promoter and enables the production of a modified promoter capable of strong expression induction and a method for efficient secretion and production of alkaline cellulase with microorganisms to which the modified promoter has been applied.

For example, PTL 2 discloses a method in which the base sequence of a promoter of the R-specific enoyl-CoA hydratase gene of a bacterium belonging to the genus Aeromonas is modified in order to prepare a modified promoter capable of controlling the expression of the above gene and a polyhydroxyalkanoate copolymer with a regulated monomer composition ratio is efficiently produced using microorganisms to which the above modified promoter has been applied.

### Citation List

### Patent Literature

PTL 1: Japanese Unexamined Patent Application Publication No. 2005-278644
PTL 2: International Publication No. 2015/115619

### Summary of Invention

### Technical Problem

Depending on the type of a substance, the amount of expression of a gene coding the substance may decrease, even when a modified promoter having a high promoter activity is used. Therefore, it is necessary to control the activity of the promoter appropriately in accordance with the type of the substance that is to be produced by microorganisms. However, it is difficult to control the activity of the promoter.

Accordingly, the present disclosure provides a modified promoter with which the amount of expression of a gene coding a substance can be controlled with flexibility, an expression vector, a microorganism, a method for producing a substance, a modified cyanobacterium, and a method for preparing a modified promoter.

### Solution to Problem

A modified promoter according to an aspect of the present disclosure is a modified promoter including a base sequence (SEQ ID NO: 1) located upstream of a start codon of a slr1841 gene, the base sequence consisting of 348 bases, or a base sequence having 80% or more homology with the base sequence represented by SEQ ID NO: 1, the base sequence being modified by (i) replacement of one or more bases included in the base sequence with another base, (ii) deletion of one or more bases included in the base sequence, or (iii) insertion of one or more other bases into the base sequence. Advantageous Effects of Invention

The modified promoter and method for producing a modified promoter according to the present disclosure can provide a modified promoter with which the amount of expression of a gene coding a substance can be controlled with flexibility. The expression vector according to the present disclosure can provide an expression vector with which the amount of expression of a gene coding a substance can be controlled with flexibility in a transformed cell. According to the microorganism, the method for producing a substance, and the modified cyanobacterium according to the present disclosure, a microorganism, a method for producing a substance, and a modified cyanobacterium with which a gene coding a substance can be expressed and the substance coded by the gene can be produced with an intended efficiency can be provided.

### Brief Description of Drawings

[Fig. 1] Fig. 1 is a schematic diagram illustrating the slr1841 promoter region.
[Fig. 2] Fig. 2 is a diagram illustrating the position of the transcription start site (TSS) of the slr1841 gene.
[Fig. 3] Fig. 3 is a flowchart illustrating an example of a method for preparing a modified promoter.
[Fig. 4] Fig. 4 is a schematic diagram illustrating an example of an expression vector.
[Fig. 5] Fig. 5 is an electrophoresis image of a fragment obtained by 5'-RACE (5'-rapid amplification of cDNA ends).
[Fig. 6] Fig. 6 is a diagram illustrating the results of measurement of the fluorescence intensities of culture solutions of transformed cyanobacteria and transformed colibacillus.
[Fig. 7A] Fig. 7A is a diagram illustrating the structures of modified promoters having mutations introduced in a region of the slr1841 promoter which is upstream of the transcription start site (TSS) and the results of measurement of the fluorescence intensities of culture solutions of transformed cells in which the modified promoters are introduced.
[Fig. 7B] Fig. 7B is a diagram illustrating the base sequences of the -10 regions in which mutations are introduced and the results of measurement of the fluorescence intensities of culture solutions of transformed cells in which modified promoters having the above mutations introduced therein are introduced.
[Fig. 8] Fig. 8 is a diagram illustrating the results of analysis of culture supernatants by LC-MS (liquid chromatograph-mass spectrometry).
[Fig. 9] Fig. 9 is a diagram illustrating the results of antibiotic resistance tests of modified and wild strains.

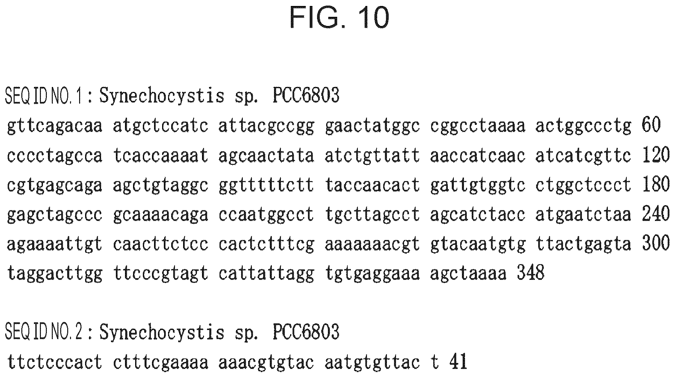

[Fig. 10] Fig. 10 illustrates SEQ ID NOs: 1 and 2.

Description of Embodiments

### (Underlying Knowledge Forming Basis of the Present Disclosure)

The production of substances with microorganisms (in particular, fermentation) has been practiced from old times. For example, in the production of useful substances by fermentation, fermented foods, alcoholic drinks, and the like which include substances useful for humans (specifically, organic substances) are produced while the organic substances incorporated by microorganisms as nutrients are anaerobically metabolized to produce energy. Nowadays, the production of substances with microorganisms has been applied to not only the conventional fermentative production as described above but also the production of various types of substances, such as amino acids, nucleic acids, antibiotics, and proteins.

A number of unknown types of microorganisms exist in the natural world. It is said that only a few percent of all the microorganisms have been discovered so far. Therefore, microorganisms are considered abundant gene resources. Accordingly, constructing a system capable of producing a useful substance with high efficiency compared with the related art by discovering a microorganism having a useful function, such as a microorganism (hereinafter, referred to as "useful microorganism") capable of increasing the efficiency of production of an intended substance, in the natural world and isolating and breeding the useful microorganism has been anticipated.

With recent advances of biotechnology, a technology of preparing a microorganism (hereinafter, referred to as "gene recombinant microorganism") implanted with a specific gene of a useful microorganism using a gene recombination technology or the like and producing a useful substance using the gene recombinant microorganism has been actively studied.

For producing a useful substance (e.g., a useful protein) using the gene recombinant microorganism, it is necessary to not only introduce a gene coding the useful substance (i.e., the useful protein) into the genome of the microorganisms but also introduce an appropriate promoter to the upstream of the gene to induce the expression of the gene.

A promoter is a region present in all the genes included in the genome (hereinafter, referred to also as "genome DNA (deoxyribonucleic acid)") present inside a cell. A promoter plays important roles, that is, a promoter appropriately induces the expression of a gene in a region upstream of the gene and controls the amount of expression (i.e., the amount of transcription) of the gene. Thus, for producing a useful substance (a useful protein) using a microorganism, it is essential to select or develop a promoter suitable for efficient transcription of a gene coding the useful substance (the useful protein).

The higher the activity of a promoter, the larger the amount of transcription of a gene located downstream thereof (specifically, a gene coding an intended useful protein). It is easy to imagine that this increases the efficiency of production of the intended useful protein. Although there are a number of similar cases as described above, it is not always true that a strong promoter be suitable for the production of all the microorganisms (also referred to as "cells") or all the proteins. If the activity of the promoter is excessively high, the amount of the protein produced may exceed the capacity of the cell and, consequently, it may become difficult to fold the protein into a normal, three-dimensional structure. In such a case, the efficient production of a useful protein cannot be achieved. This may also impair the growth of the cell.

Thus, the development of a promoter such that, in the case where a useful protein is produced using a gene recombinant microorganism, the activity of the promoter which induces the expression of the protein can be flexibly controlled with consideration of the balance between the capacity and production efficiency of the microorganism is anticipated. It is considered that the development of the above-described promoter makes it easy to select a promoter suitable for the production of an intended protein and enables the efficient production of the protein.

However, as for microorganisms the promoters of which are hardly known in the related art, such as a cyanobacterium, promoters that can be flexibly used for producing proteins using the microorganism have not been developed (NPL 1: Marnix H. Medema et al., "Synthetic biology in streptomyces bacteria", Methods in Enzymology, Volume 497, 2011, Pages 485-502, ISSN: 0076-6879).

The inventors of the present invention conducted extensive studies and consequently focused on the promoter of the slr1841 gene, which is one of the most expressed proteins in a cyanobacterium. The inventors of the present invention identified a base sequence particularly important for exhibiting the function of the promoter and found that the activity of the promoter can be changed from a few percent to about 280 percent by modifying the base sequence thereof (i.e., modified promoter). This enables, for example, promoters suitable for controlling the amounts of expression of genes of various microorganisms, such as a cyanobacterium, to be easily selected. For example, applying the modified promoter according to the present disclosure to a cyanobacterium reduces the amount of expression of the genes (e.g., slr1841 gene) that cyanobacteria originally hold to an intended level. This also enables the deprivation of the outer membrane to occur to a degree at which the cyanobacterium is capable of producing a substance while maintaining the life activity.

Accordingly, the present disclosure provides a modified promoter such that the activity of the promoter which induces the expression of an intended protein can be flexibly controlled by modifying the gene sequence (i.e., base sequence) of a promoter derived from a cyanobacterium. The present disclosure also provides an expression vector with which the amounts of expression of intended substances (e.g., a protein) in various microorganisms, such as a cyanobacterium, can be controlled. The present disclosure further provides a method for producing a substance (i.e., a useful substance) using the microorganism with efficiency.

### (Summary of the Present Disclosure)

The summary of an aspect of the present disclosure is as follows.

A modified promoter according to an aspect of the present disclosure is a modified promoter including a base sequence (SEQ ID NO: 1) located upstream of a start codon of a slr1841 gene, the base sequence consisting of 348 bases, or a base sequence having 80% or more homology with the base sequence represented by SEQ ID NO: 1, the base sequence being modified by at least one selected from the group consisting of (i) replacement of one or more bases included in the base sequence with another base, (ii) deletion of one or more bases included in the base sequence, and (iii) insertion of one or more bases into the base sequence.

This enables the modified promoter to flexibly control the amount of expression of a gene located downstream of the modified promoter.

For example, a modified promoter according to an aspect of the present disclosure is a modified promoter in which, in the base sequence represented by SEQ ID NO: 1, a region of a base sequence (SEQ ID NO: 2) extending from 54 to 94 bases upstream of the start codon, or a region corresponding to the region of the base sequence represented by SEQ ID NO: 2, is modified by at least one selected from the group consisting of (i) replacement of one or more bases included in the region with another base, (ii) deletion of one or more bases included in the region, and (iii) insertion of one or more bases into the region.

In the above modified promoter, since a base sequence region represented by SEQ ID NO: 2, which is the core region of the slr1841 promoter, or a region corresponding to the base sequence region is modified, the promoter activity is likely to be further affected. Accordingly, the modified promoter can control the amount of expression of a gene located downstream of the modified promoter in a further flexible manner.

A modified promoter according to an aspect of the present disclosure is a modified promoter in which, in the base sequence represented by SEQ ID NO: 1, a region of a base sequence TACAAT extending from 62 to 67 bases upstream of the start codon, or a region corresponding to the region of the base sequence TACAAT, is modified by at least one selected from the group consisting of (i) replacement of one or more bases included in the region with another base, (ii) deletion of one or more bases included in the region, and (iii) insertion of one or more bases into the region.

In the above modified promoter, since the base sequence TACAAT region, which is the -10 region of the promoter of the slr1841 gene, or a region corresponding to the above region is modified, the binding specificity between the general transcription factor and the promoter can be readily changed. This makes it easy to control the promoter activity of the modified promoter. Accordingly, the amount of expression of a gene located downstream of the modified promoter can be controlled in a further flexible manner.

A modified promoter according to an aspect of the present disclosure is a modified promoter in which, in the base sequence represented by SEQ ID NO: 1, a region of a base sequence TTCTCC extending from 89 to 94 bases upstream of the start codon, or a region corresponding to the region of the base sequence TTCTCC , is modified by at least one selected from the group consisting of (i) replacement of one or more bases included in the region with another base, (ii) deletion of one or more bases included in the region, and (iii) insertion of one or more bases into the region.

In the above modified promoter, since the base sequence TTCTCC region, which is the -35 region of the promoter of the slr1841 gene, or a region corresponding to the above region is modified, the binding specificity between the general transcription factor (i.e., transcription initiation factor) and the promoter can be readily changed. This makes it easy to control the promoter activity of the modified promoter. Accordingly, the amount of expression of a gene located downstream of the modified promoter can be controlled in a further flexible manner.

An expression vector according to an aspect of the present disclosure includes any one of the above-described modified promoters.

This enables the expression vector to cause a gene located downstream of the modified promoter to be expressed in a transformed cell with an intended expression efficiency.

A microorganism according to an aspect of the present disclosure includes the above-described expression vector.

The above microorganism can produce a substance coded by the gene expressed by the above expression vector with an intended efficiency.

A microorganism according to an aspect of the present disclosure includes any one of the above-described modified promoters introduced to a genome.

As a result of expression of a gene located downstream of the modified promoter, the above microorganism can produce a substance coded by the gene with an intended efficiency.

A modified cyanobacterium according to an aspect of the present disclosure includes any one of the above-described modified promoters introduced to a genome.

Since the above modified cyanobacterium includes a modified promoter which enables the amount of expression of the slr1841 gene to be flexibly controlled, the modified promoter being introduced in the genome, Slr1841 can be expressed in an intended amount. For example, when a modified promoter the activity of which has been reduced to a degree at which the life activity can be maintained is introduced into the genome of the modified cyanobacterium, deprivation of the outer membrane occurs while the life activity is maintained and, consequently, the likelihood of metabolic substances produced inside the cell leaking out of the cell is increased.

A method for producing a substance according to an aspect of the present disclosure includes cultivating a microorganism including the above-described expression vector or a microorganism including a genome and any one of the above-described modified promoters introduced to the genome to cause the microorganism to produce a substance.

According to the above method for producing a substance, since a substance can be produced by cultivating a microorganism, it is possible to produce a substance with high efficiency.

A method for producing a substance according to an aspect of the present disclosure includes cultivating the above-described modified cyanobacterium to cause the modified cyanobacterium to produce a substance.

According to the above method for producing a substance, since a substance can be produced by cultivating a modified cyanobacterium, it is possible to produce a substance with high efficiency.

A method for preparing a modified promoter according to an aspect of the present disclosure includes modifying a base sequence (SEQ ID NO: 1) upstream of a start codon of a slr1841 gene, the base sequence consisting of 348 bases, or a base sequence having 80% or more homology with the base sequence represented by SEQ ID NO: 1, by at least one selected from the group consisting of (i) replacement of one or more bases included in the base sequence with another base, (ii) deletion of one or more bases included in the base sequence, and (iii) insertion of one or more bases into the base sequence.

Using the method for preparing a modified promoter, a modified promoter with which the amount of expression of a gene located downstream of the modified promoter can be flexibly controlled can be prepared.

Embodiments are described specifically with reference to the attached drawings below.

It should be noted that the embodiments described below are all comprehensive or specific examples. The values, materials, steps, the order of the steps, and the like described in the following embodiments are intended to be illustrative but not restrictive of the present disclosure. Among the elements described in the following embodiments, the elements not described in the independent claims, which indicate the highest concept, are described as optional elements.

The attached drawings are not always exactly drawn. In the drawings, substantially the same structures are denoted by the same reference numeral, and the duplicate description thereof may be omitted or simplified.

Hereinafter, a numerical range means not only a strict meaning but also, for example, a substantially equivalent range, such as the amount (e.g., number or concentration) of expression of a protein, or measuring the above range.

The term "cell" used herein refers to a microorganism (e.g., a cyanobacterium).

### (Embodiments)

### [1. Definitions]

In the description, the homology between base sequences or amino acid sequences is calculated using the BLAST (basic local alignment search tool) algorithm. Specifically, the above homology is calculated by a pairwise analysis using the BLAST program available in the website (https://blast.ncbi.nlm.nih.gov/Blast.cgi) of NCBI (National Center for Biotechnology Information). Information regarding the base sequences of genes and information regarding the amino acid sequences of proteins coded by the genes are published on the NCBI website and the like and available anytime.

A promoter is a region that serves as a transcription start site upon the transcription of a gene from a genome. In other words, a promoter is a control sequence (control region) responsible for the efficiency of the transcription initiation reaction of a gene. The term "promoter" refers broadly to both control sequence (control region) that acts at a position relatively close to the transcription start site and control sequence (control region) that acts at a position distant from the transcription start site. For example, in eubacteria, a promoter commonly includes a sequence that extends from the -35 region, which is 35 bases upstream of the transcription start site of a gene, to the -10 region, which is 10 bases upstream of the transcription start site of a gene. These regions have various sequences. For example, it is known that, in colibacillus, the consensus sequences of the - 35 and -10 regions are, for example, TTGACA and TATAAT, respectively (NPL 2: Calvin B. Harley et al., "Analysis of E. coli promoter sequences", Nucleic Acid Research, Volume 15, Issue 5, 11 March 1987, Pages 2343-2361, https://doi.org/10.109 3/nar/15.5.2343).

### [2. Modified Promoter]

A modified promoter according to an embodiment is described below. The modified promoter according to the embodiment is a promoter including the base sequence of a promoter (hereinafter, referred to also as "slr1841 promoter") of the slr1841 gene present in the genome DNA of a cyanobacterium, the base sequence being modified by replacement, deletion, or insertion of one or more bases. The promoter activity of the above modified promoter can be controlled in a flexible manner.

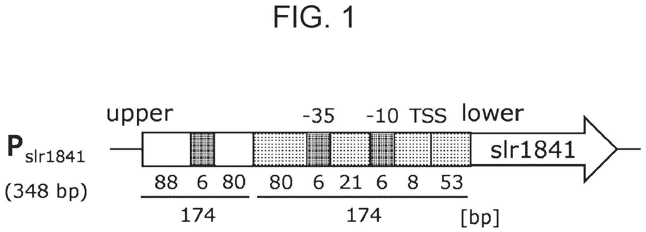

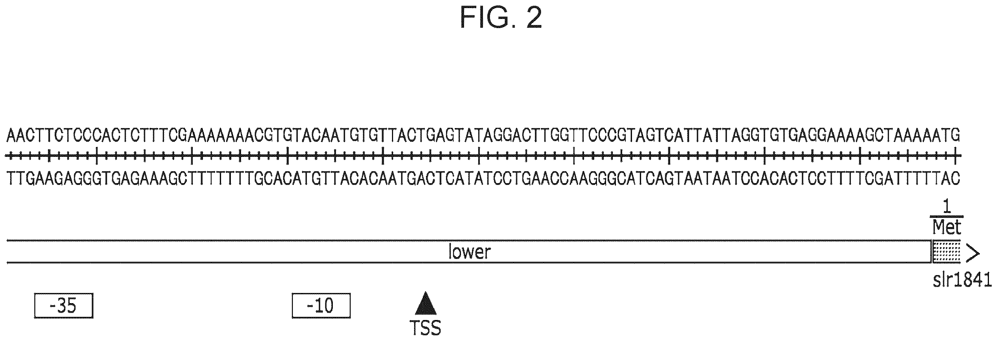

The slr1841 promoter is described below. Fig. 1 is a schematic diagram illustrating the slr1841 promoter region. Fig. 2 is a diagram illustrating the position of the transcription start site (TSS) of the slr1841 gene.

As illustrated in Fig. 1, the slr1841 promoter region is a base sequence region (SEQ ID NO: 1) that is located upstream of the start codon of the slr1841 gene and consists of 348 bases. The slr1841 promoter region plays an important role in controlling the timing and amount of expression of the slr1841 gene. There has been little knowledge about the base sequence of the slr1841 promoter of cyanobacteria, the activity of the promoter, and the like. Accordingly, the present inventors analyzed the base sequence of the slr1841 promoter by 5'-RACE (5'-rapid amplification of cDNA ends).

As illustrated in Fig. 2, the transcription start site (TSS) of the slr1841 gene is located 54 bases upstream of the start codon. A region that extends from the transcription start site to the -35 region is referred to as "core region (i.e., core promoter)", which is specifically a base sequence region (SEQ ID NO: 2) that extends from 54 to 94 bases upstream of the start codon. The -35 region of the slr1841 promoter is a region of base sequence TTCTCC which extends from 89 to 94 bases upstream of the start codon. The - 10 region of the slr1841 promoter is a region of base sequence TACAAT which extends from 62 to 67 bases upstream of the start codon.

The base sequence of the promoter that is to be modified may be a base sequence having 80% or more homology, is preferably a base sequence having 90% or more homology, is more preferably a base sequence having 95% or more homology, and is particularly preferably a base sequence having 98% or more homology with the base sequence (SEQ ID NO: 1) of the slr1841 promoter.

The slr1841 gene is a gene coding Slr1841, which is one of the most expressed proteins in the cells of cyanobacteria. Slr1841 is an SLH domain-containing outer membrane protein and includes a C-end-side region buried in a lipidic membrane (also referred to as "outer membrane") and an N-end-side SLH domain protruded from the lipidic membrane (i.e., the outer membrane). Slr1841 is broadly distributed in cyanobacteria and germs belonging to Negativicutes, which is a group of Gram-negative bacteria. The region of the SLH domain-containing protein which is buried in a lipidic membrane (i.e., an outer membrane) forms a channel through which hydrophilic substances can permeate the outer membrane, while the SLH domain is capable of binding to cell walls. Examples of genes coding the SLH domain-containing outer membrane protein include slr1841 and slr1908 held by Synechocystis sp. PCC 6803 and oprB held by Anabaena sp. 90.

The promoter is not limited and may be any promoter of a gene held by eubacteria the expression of which can be induced regularly and strongly. Examples of the promoter include the base sequences of promoters that induce a series of proteins most expressed in cells, such as slr1841, slr0688, and slr1908, which are membrane proteins held by cyanobacterium Synechocystis sp. PCC 6803 and regions corresponding to the base sequences of the above promoters, which are held by cyanobacterium Synechococcus, Anabaena, and Nostoc.

### [3. Preparation (Construction) of Modified Promoter]

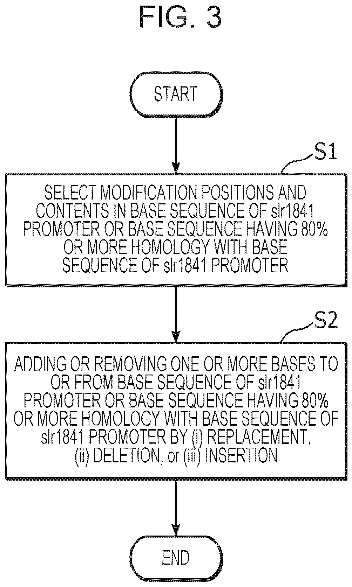

The modified promoter may be prepared in the following manner. Fig. 3 is a flowchart illustrating an example of a method for preparing a modified promoter.

As illustrated in Fig. 3, the position at which the base sequence (SEQ ID NO: 1) of the slr1841 promoter or a base sequence having 80% or more homology with the base sequence (SEQ ID NO: 1) of the slr1841 promoter is to be modified and the contents of the modification are selected (Step S1).

For example, the above modification position may be any position within the promoter region. The modification method is also not limited. For example, the above modification position may be within the base sequence region represented by SEQ ID NO: 1 (i.e., the slr1841 promoter region), is preferably within the base sequence region (i.e., core region) represented by SEQ ID NO: 2, which corresponds to the main body of the promoter, and is more preferably within the base sequence TACAAT region corresponding to the -10 region or the base sequence TTCTCC region corresponding to the -35 region. In such a case, the impacts on promoter activity can be increased.

Subsequently, the base sequence (SEQ ID NO: 1) of the slr1841 promoter or a base sequence having 80% or more homology with the base sequence (SEQ ID NO: 1) of the slr1841 promoter is modified by (i) substitution, (ii) deletion, or (iii) insertion of one or more bases (Step S2). Hereby, a modified promoter having a promoter region with a modified base sequence is prepared.

The modification position may be within a base sequence region having 80% or more homology with any of the above base sequences. For example, the above modification may be performed by (i) replacement of one or more (e.g., about one to three) bases included in the base sequence of the promoter with other bases, (ii) deletion of one or more (e.g., about one to three) bases included in the base sequence of the promoter, or (iii) insertion of one or more (e.g., about one to three) bases into the base sequence of the promoter.

As described above, the modified promoter can be constructed by artificial gene synthesis. The means for construction is not limited. For example, the modified promoter can be constructed by extracting a genome DNA from a cell (e.g., a cyanobacterium), amplifying the base sequence of an intended promoter region (e.g., slr1841 promoter region) in the genome DNA by PCR (polymerase chain reaction), introducing the resulting gene fragment into a plasmid, and subsequently performing modification. Site directed mutagenesis is a method useful for introducing a displacement to a specific site of a plasmid and enables the replacement, deletion, and insertion of genes. The introduction of the above mutations is commonly performed by inverse PCR. The use of a site directed mutagenesis kit, such as "PrimeSTAR(registered trademark) Mutagenesis Basal Kit" produced by Takara Bio Inc. or "KOD -Plus-Mutagenesis Kit" produced by Toyobo Co., Ltd., facilitates the above process.

### [4. Determination of Activity of Modified Promoter]

The activity of the modified promoter can be determined by introducing, in a cell, an expression vector that includes the modified promoter and a base sequence added downstream of the modified promoter, the base sequence coding a protein, and analyzing the amount of expression of the protein. The type of the cell into which the expression vector is to be introduced and the method by which the expression vector is introduced into the cell are not limited. It is easy and simple to introduce the expression vector into a commercial colibacillus cell by a heat shock method. The type of the protein the gene expression of which is induced by the modified promoter is not limited. The protein may be a protein having a relatively stable three-dimensional structure, such as green fluorescent protein (GFP). The method for analyzing the amount of expression of a protein is also not limited. If the protein is GFP, the amount of expression of GFP can be determined by measuring the intensity of fluorescence emitted by the cell or analyzing all the proteins extracted from the cell with a GFP tag antibody by an Western Blotting method.

### [5. Example of Application of Modified Promoter]

Examples of application of the modified promoter are efficient production of useful substances with microorganisms, promotion of secretion of intracellular products, improvement of drug resistance, and improvement of environment resistance. Note that examples of application of the modified promoter are not limited thereto. The microorganisms are described below.

Examples of the useful substances and the intracellular products include a protein, an amino acid, a nucleic acid-related substance, and an antibiotic. The method for producing a substance may be, but not limited to, a method including cultivating a microorganism to cause the microorganism to secrete an intended substance (useful substance or intracellular product) outside the cell and appropriately isolating the intended substance from the culture solution. For appropriately isolating the intended substance from the culture solution, for example, a membrane permeable to the intended substance may be used, and a substance that passes through the permeable membrane may be collected. Since the intended substance secreted in the culture solution can be collected while the microorganism is cultured, it becomes unnecessary to remove the microorganism from the culture solution. This makes it possible to produce the intended substance with further ease, efficiency, and simplicity.

### [6. Expression Vector]

An expression vector according to an embodiment includes a modified promoter. Specifically, the expression vector is a plasmid or virus designed for expression of genes in cells. For example, the expression vector includes a gene fragment introduced in the plasmid, the gene fragment including a modified promoter and a base sequence coding a protein, the base sequence being located downstream of the modified promoter.

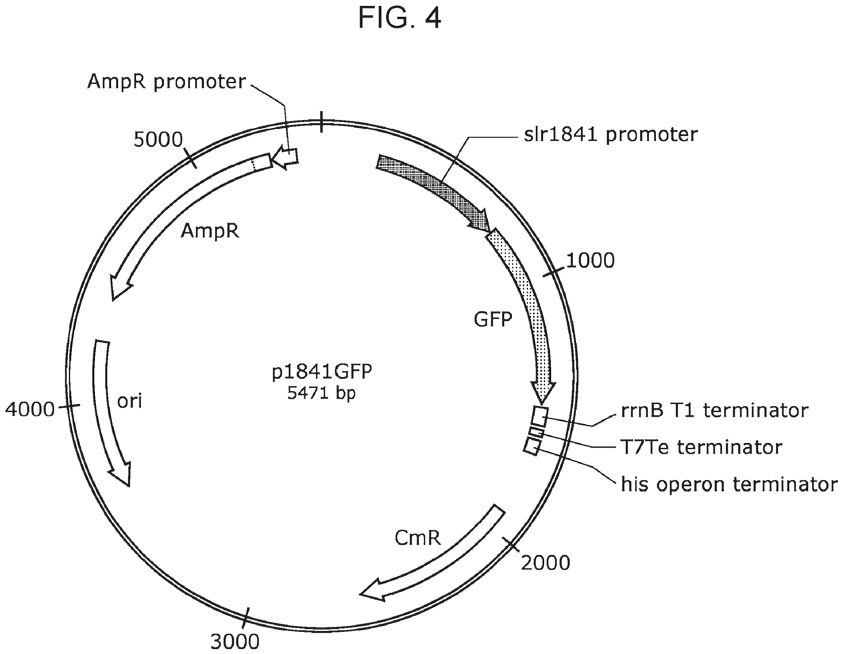

Fig. 4 is a schematic diagram illustrating an example of the expression vector. In Fig. 4, the protein the expression of which is controlled by the modified promoter is GFP. The expression vector illustrated in Fig. 4 is constructed as a result of a gene coding GFP being inserted in the plasmid at a position downstream of the slr1841 promoter. The activity of the modified promoter can be determined by introducing the expression vector into colibacillus and measuring the fluorescence emitted by GFP expressed in the transformed cells.

The location in which the modified promoter is designed and constructed is not limited to the outside of a microorganism cell, unlike the construction of the expression vector or the like. The modified promoter may be constructed by directly modifying an intended promoter included in the genome DNA of a cell, and the modified promoter may be caused to act inside the cell. The method for modifying the genome DNA is not limited; any of gene recombination, genome editing, and mutagenesis may be used. Examples of the method for modifying the genome DNA of cyanobacteria that may be used include gene recombination by natural transformation (NPL 3: Yuu Hirose et al., Photosynthesis Study, Low Temperature Science, Volume 67, Institute of Low Temperature Science, Hokkaido University, 2008, pp. 9-15, ISSN: 1880-7593, http://hdl.handle.net/2115/39084) and genome editing using the CRISPR-Cpf1 system (NPL 4: Justin Ungerer et al., "Cpf1 Is A Versatile Tool for CRISPR Genome Editing Across Diverse Species of Cyanobacteria", Scientific Reports, Volume 6, 2016, Article number: 39681).

### [7. Microorganism]

A microorganism according to an embodiment may include an expression vector including a modified promoter in the cell and may include a modified promoter introduced in the genome. Examples of the microorganism that may be used include, but are not limited to, eubacteria, such as colibacillus, bacillus subtilis, and cyanobacterium, which are relatively easy to handle. If the microorganism is a cyanobacterium, a cyanobacterium including a modified promoter introduced in the genome is referred to as "modified cyanobacterium". The above microorganisms are capable of exhibiting various functions depending on the type of the gene inserted downstream of the modified promoter. For example, the productivity with which the microorganisms produce substances may be enhanced. In another case, the defense functions of the cells, such as drug resistance and environment resistance, may be enhanced.

### [8. Advantageous Effects, etc.]

As described above, the modified promoter according to the embodiment is a modified promoter including a base sequence (SEQ ID NO: 1) located upstream of a start codon of a slr1841 gene, the base sequence consisting of 348 bases, or a base sequence having 80% or more homology with the base sequence represented by SEQ ID NO: 1, the base sequence being modified by at least one selected from the group consisting of (i) replacement of one or more bases included in the base sequence with another base, (ii) deletion of one or more bases included in the base sequence, and (iii) insertion of one or more bases into the base sequence.

This enables the modified promoter to flexibly control the amount of expression of a gene located downstream of the modified promoter.

For example, the modified promoter according to the embodiment is a modified promoter in which, in the base sequence represented by SEQ ID NO: 1, a region of a base sequence (SEQ ID NO: 2) extending from 54 to 94 bases upstream of the start codon, or a region corresponding to the region of the base sequence represented by SEQ ID NO: 2, is modified by at least one selected from the group consisting of (i) replacement of one or more bases included in the region with another base, (ii) deletion of one or more bases included in the region, and (iii) insertion of one or more bases into the region.

In the above modified promoter, since a base sequence region represented by SEQ ID NO: 2, which is the core region of the slr1841 promoter, or a region corresponding to the base sequence region is modified, the promoter activity is likely to be further affected. Accordingly, the modified promoter can control the amount of expression of a gene located downstream of the modified promoter in a further flexible manner.

The modified promoter according to the embodiment is a modified promoter in which, in the base sequence represented by SEQ ID NO: 1, a region of a base sequence TACAAT extending from 62 to 67 bases upstream of the start codon, or a region corresponding to the region of the base sequence TACAAT, is modified by at least one selected from the group consisting of (i) replacement of one or more bases included in the region with another base, (ii) deletion of one or more bases included in the region, and (iii) insertion of one or more bases into the region.

In the above modified promoter, since the base sequence TACAAT region, which is the -10 region of the promoter of the slr1841 gene, or a region corresponding to the above region is modified, the binding specificity between the general transcription factor and the promoter can be readily changed. This makes it easy to control the promoter activity of the modified promoter. Accordingly, the amount of expression of a gene located downstream of the modified promoter can be controlled in a further flexible manner.

The modified promoter according to the embodiment is a modified promoter in which, in the base sequence represented by SEQ ID NO: 1, a region of a base sequence TTCTCC extending from 89 to 94 bases upstream of the start codon, or a region corresponding to the region of the base sequence TTCTCC , is modified by at least one selected from the group consisting of (i) replacement of one or more bases included in the region with another base, (ii) deletion of one or more bases included in the region, and (iii) insertion of one or more bases into the region.

In the above modified promoter, since the base sequence TTCTCC region, which is the -35 region of the promoter of the slr1841 gene, or a region corresponding to the above region is modified, the binding specificity between the general transcription factor (i.e., transcription initiation factor) and the promoter can be readily changed. This makes it easy to control the promoter activity of the modified promoter. Accordingly, the amount of expression of a gene located downstream of the modified promoter can be controlled in a further flexible manner.

The expression vector according to the embodiment includes any one of the above-described modified promoters.

This enables the expression vector to cause a gene located downstream of the modified promoter to be expressed in a transformed cell with an intended expression efficiency.

The microorganism according to the embodiment includes the above-described expression vector.

The above microorganism can produce a substance coded by the gene expressed by the above expression vector with an intended efficiency.

The microorganism according to the embodiment includes any one of the above-described modified promoters introduced to a genome.

As a result of expression of a gene located downstream of the modified promoter, the above microorganism can produce a substance coded by the gene with an intended efficiency.

The modified cyanobacterium according to the embodiment includes any one of the above-described modified promoters introduced to a genome.

Since the above modified cyanobacterium includes a modified promoter which enables the amount of expression of the slr1841 gene to be flexibly controlled, the modified promoter being introduced in the genome, Slr1841 can be expressed in an intended amount. For example, when a modified promoter the activity of which has been reduced to a degree at which the life activity can be maintained is introduced into the genome of the modified cyanobacterium, deprivation of the outer membrane occurs while the life activity is maintained and, consequently, the likelihood of metabolic substances produced inside the cell leaking out of the cell is increased.

The method for producing a substance according to the embodiment includes cultivating a microorganism including the above-described expression vector or a microorganism including a genome and any one of the above-described modified promoters introduced to the genome to cause the microorganism to produce a substance.

In the method for producing a substance, since a substance can be produced by cultivating a microorganism, it is possible to produce an intended substance with high efficiency.

The method for producing a substance according to the embodiment includes cultivating the above-described modified cyanobacterium to cause the modified cyanobacterium to produce a substance.

In the method for producing a substance, since a substance can be produced by cultivating a modified cyanobacterium, it is possible to produce a substance with high efficiency.

The method for preparing a modified promoter according to the embodiment includes modifying a base sequence (SEQ ID NO: 1) upstream of a start codon of a slr1841 gene, the base sequence consisting of 348 bases, or a base sequence having 80% or more homology with the base sequence represented by SEQ ID NO: 1, by at least one selected from the group consisting of (i) replacement of one or more bases included in the base sequence with another base, (ii) deletion of one or more bases included in the base sequence, and (iii) insertion of one or more bases into the base sequence.

Using the method for preparing a modified promoter, a modified promoter with which the amount of expression of a gene located downstream of the modified promoter can be flexibly controlled can be prepared.

### Examples

The modified promoter, expression vector, microorganism, method for producing a substance, modified cyanobacterium, and method for preparing a modified promoter according to the present disclosure are described specifically with refence to Examples below. It should be noted that the present disclosure is not limited by Examples below.

### (Example 1) Determination of Sequence of slr1841 Promoter

In Example 1, the sequence of a promoter of the slr1841 gene coding the SLH domain-containing outer membrane protein was determined.

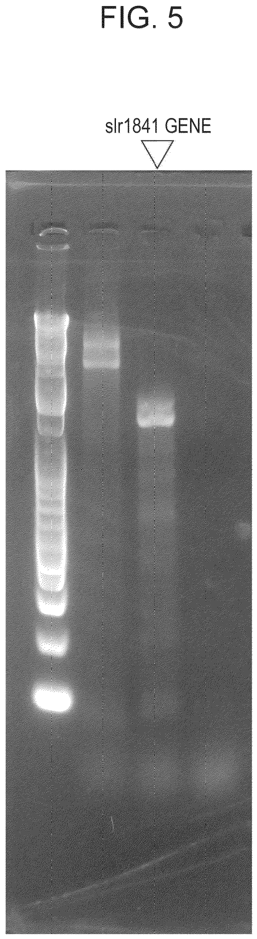

Specifically, first, the total RNA (ribonucleic acid) was extracted from the cell of a cyanobacterium Synechocystis sp. PCC 6803. Subsequently, a cDNA was synthesized from the total RNA. After the ends of the cDNA had been blunted, an adapter was bonded thereto. Hereby, a PCR library was prepared. The above cDNA was diluted and used as a template. A fragment obtained by 5'-RACE was subcloned into a T-vector. Then, the sequence was analyzed. Fig. 5 is an electrophoresis image of the fragment obtained by 5'-RACE. The image on the far left in Fig. 5 is an electrophoresis image of a molecular weight marker ("Gene Ladder Wide 2" produced by Nippon Gene Co., Ltd.). The second image from the left is an electrophoresis image of the control ("SMARTer (registered trademark) RACE 5'/3' Kit" produced by Clontech Laboratories, Inc.). As illustrated in Fig. 5, it was confirmed that the amplified fragment of the slr1841 gene was obtained by 5'-RACE.

On the basis of the results of the sequence analysis, the base sequence (SEQ ID NO: 1) of the slr1841 promoter was determined. The transcription start site (TSS) of the slr1841 gene was determined to be the position 54 bases upstream of the start codon (see Figs. 1 and 2). It was considered that a region of the base sequence TTCTCC which extended 89 to 94 bases upstream of the start codon be the -35 region and that a region of the base sequence TACAAT which extended 62 to 67 bases upstream of the start codon be the -10 region.

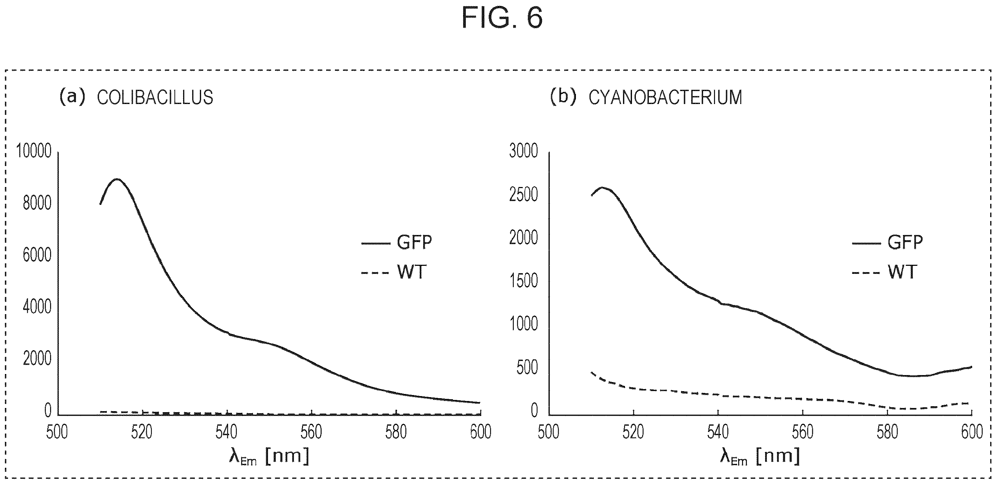

Subsequently, a plasmid DNA including the sequence (SEQ ID NO: 1) extending from the start codon to a position 348 bases upstream of the start codon, which included the main body of the promoter located upstream of the transcription start site (TSS), was prepared. Moreover, a DNA sequence coding the gene of green fluorescent protein (GFP) was introduced immediately downstream thereof (see Fig. 3). The GFP expression vector including the slr1841 promoter, which was constructed in the above-described manner, was transfected into the cell of colibacillus Escherichia coli HST08 and the cell of cyanobacterium Synechocystis sp. PCC 6803. The above cells were cultivated and whether or not they emit fluorescence was determined. Fig. 6 illustrates the results.

Fig. 6 is a diagram illustrating the results of measurement of the fluorescence intensities of culture solutions of transformed cyanobacterium and colibacillus. Fig. 6(a) illustrates the results of measurement of the fluorescence intensities of culture solutions of untransformed colibacillus (in Fig. 6(a), WT) and transformed colibacillus (in Fig. 6(a), GFP). Fig. 6(b) illustrates the results of measurement of the fluorescence intensities of culture solutions of untransformed cyanobacterium (in Fig. 6(b), WT) and transformed cyanobacterium (in Fig. 6(b), GFP). In the measurement of fluorescence intensity, the fluorescence intensity of GFP irradiated with excitation light having a wavelength of 488 nm was measured with a spectrofluorometer. As illustrated in Figs. 6(a) and 6(b), it was confirmed that the transformed cells of both colibacillus and cyanobacterium emitted fluorescence.

### (Example 2) Preparation of Modified Promoter

In Example 2, a modified promoter was prepared using the slr1841 promoter prepared in Example 1.

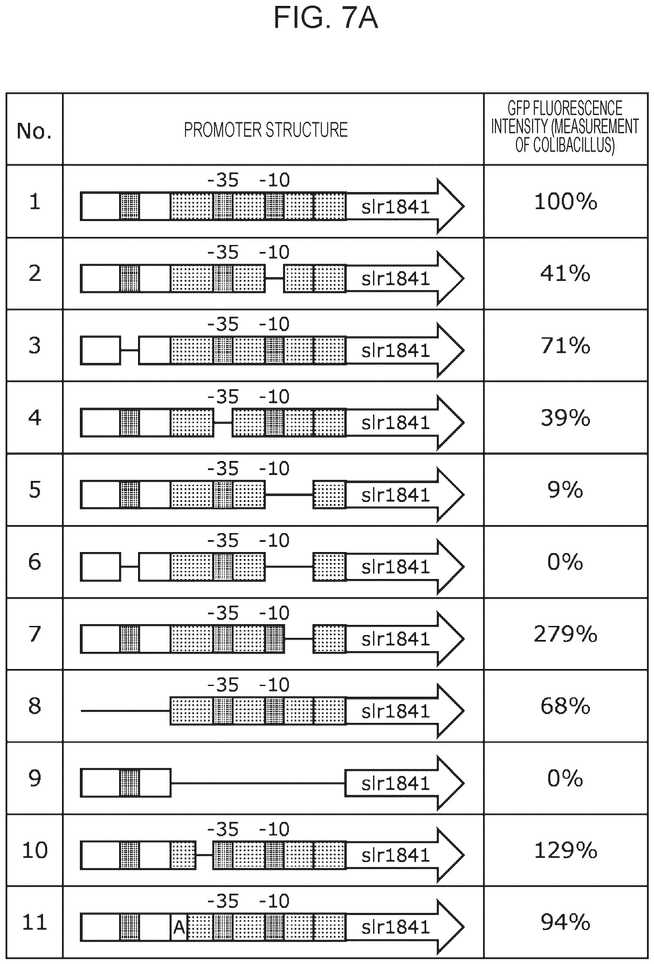

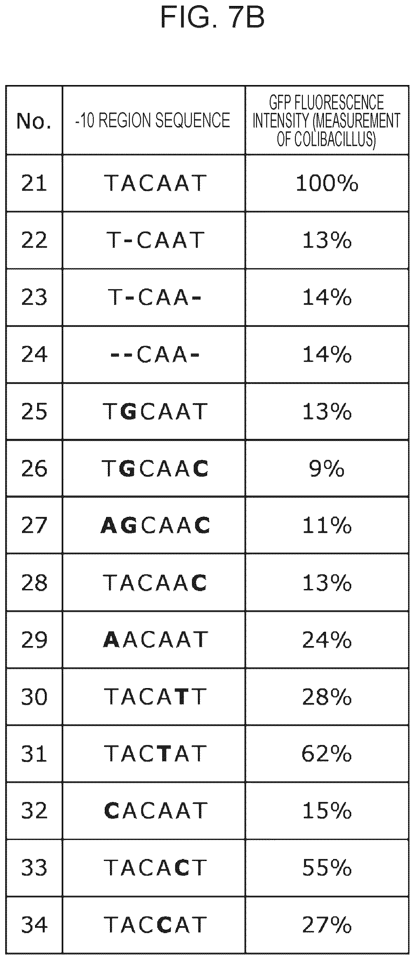

Specifically, a mutation was introduced into a plasmid including the slr1841 promoter prepared in Example 1 by an inverse PCR method. As for the position into which the mutation was introduced, the main body of the promoter which is located upstream of the transcription start site (TSS) and, in particular, the portions considered as the -10 and -35 regions were focused on. A mutation was introduced to various positions, such as positions in the vicinity of the above portions, by replacement or deletion of one to several hundred bases. GFP expression vectors including the modified promoters prepared in the above-described manner were each transfected into a cell of colibacillus Escherichia coli HST08, and the activity of the promoter was evaluated on the basis of comparison with the transformed cell of colibacillus prepared in Example 1 in terms of fluorescence intensity. Note that the fluorescence intensity of a transformed cell is the fluorescence intensity of a culture solution of the transformed cell, which is the average of the fluorescence intensities of GFP irradiated with excitation light having a wavelength of 488 nm which were measured 3 times at 525 nm with a spectrofluorometer. That is, in Example 2, the activity of each of the promoters was evaluated on the basis of comparison between the fluorescence intensity of the culture solution of the transformed cell and the fluorescence intensity (525 nm) of the culture solution of the transformed cell of colibacillus prepared in Example 1. Figs. 7A and 7B illustrate the results. Fig. 7A is a diagram illustrating the structures of modified promoters each prepared by introducing a mutation to a region of the slr1841 promoter which was located upstream of the transcription start site (TSS) and the measurement results of the fluorescence intensities of culture solutions of the transformed cells in which the modified promoters were introduced. Fig. 7B is a diagram illustrating the base sequences of the -10 regions in which mutations were introduced and the measurement results of the fluorescence intensities of culture solutions of the transformed cells in which the modified promoters, in which the mutations were introduced, were introduced. Note that No. 1 illustrated in Fig. 7A and No. 21 illustrated in Fig. 7B are the results obtained in the case where an unmodified promoter, that is, an original promoter (i.e., the slr1841 promoter) was introduced to colibacillus.

As illustrated in Fig. 7A, if mutations were introduced to the main bodies of the promoters which were located upstream of the transcription start site (TSS), various modified promoters having a promoter activity of several to about 280 percent were prepared. Moreover, as illustrated in Fig. 7B, introducing mutations to the -10 region also enables the production of various modified promoters having a promoter activity of several to about 60 percent.

### (Example 3) Genome Modification of Cyanobacterium

In Example 3, the base sequence of the slr1841 promoter included in the genome of a cyanobacterium was modified such that the structure of a modified promoter prepared in Example 2, in which a mutation that reduced the promoter activity was introduced, was achieved. Consequently, a modified cyanobacterium in which the amount of expression of the SLH domain-containing outer membrane protein by the cyanobacterium was reduced and deprivation of the outer membrane occurred was prepared.

Specifically, some of the mutations introduced in the modified promoters prepared in Example 2 which reduced the activities of the promoters were introduced into the slr1841 promoter region originally present in the genome DNA of a cyanobacterium Synechocystis sp. PCC 6803 by genome editing using CRISPR-Cpf1. The mutations introduced to the genome DNA of cyanobacterium were the mutation introduced to the slr1841 promoter in No. 2 illustrated in Fig. 7A, the mutation introduced to the -10 region in No. 29 illustrated in Fig. 7B, and the mutation introduced to the -10 region in No. 34 illustrated in Fig. 7B.

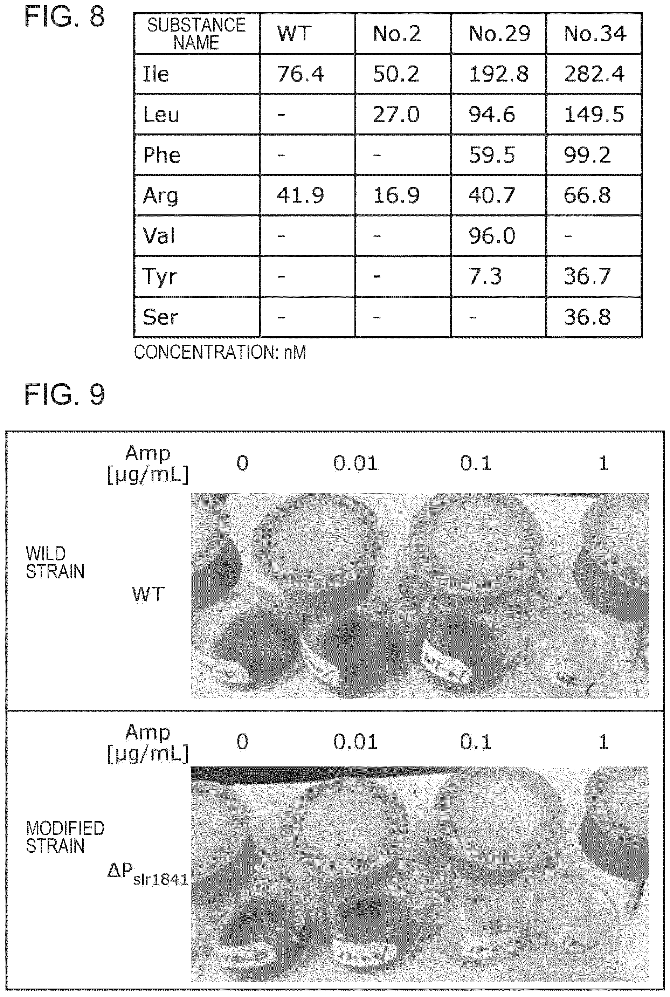

The modified cyanobacteria (hereinafter, referred to as "No. 2, 29, and 34 strains") prepared by introduction of the above mutations and cyanobacterium Synechocystis sp. PCC 6803 (hereinafter, referred to as "WT strain") were cultivated. The concentrations of amino acids in the culture supernatant of each of the cells were determined by LC-MS (liquid chromatograph-mass spectrometry). Fig. 8 illustrates the analysis results. Fig. 8 is a diagram illustrating the results of analysis of culture supernatants by LC-MS.

As illustrated in Fig. 8, the concentrations of seven types of amino acids, that is, isoleucine (Ile), leucine (Leu), phenylalanine (Phe), arginine (Arg), valine (Val), tyrosine (Tyr), and serine (Ser), were determined. The number of types of amino acids included in the culture supernatant of any of the No. 2, 29, and 34 strains was larger than that of the WT strain.

In the No. 34 strain, where the promoter activity was 27% (see Fig. 7B), the number of types of amino acids leaked outside the cells was the largest among the three modified cyanobacteria prepared in Example 3 and the concentrations of all the amino acids were higher than in the WT strain.

In the No. 29 strain, where the promoter activity was 24% (see Fig. 7B), the number of types of amino acids leaked outside the cells was the largest similarly to No. 34, but the concentration of arginine (Arg) was substantially equal to that in the WT strain.

In the No. 2 strain, where the promoter activity was 41% (see Fig. 7A), the number of types of amino acids leaked outside the cells was the smallest among the three modified cyanobacteria prepared in Example 3 but was larger than that of the WT strain by one.

Subsequently, an antibiotic ampicillin (concentration: 0 µg/mL, 0.01 µg/mL, 0.1 µg/mL, or 1 µg/mL) was added to a medium of the modified cyanobacterium in which deprivation of the outer membrane occurred (hereinafter, referred to as "modified strain") and a medium of cyanobacterium Synechocystis sp. PCC 6803 (hereinafter, referred to as "wild strain") in order to determine the antibiotic resistance of the modified strain and the wild strain. Note that the above modified strain is the No. 28 strain, which was prepared by introducing the modified promoter described as No. 28 in Fig. 7B into the genome DNA. Fig. 9 illustrates the results. Fig. 9 is a diagram illustrating the results of antibiotic resistance tests of the modified and wild strains.

As illustrated in Fig. 9, when 0.1 µg/mL of anti-ampicillin was added to the media, the modified strain became extincted, while the wild strain grows without no incident. The above results indicated that, when the amount of expression of slr1841 is reduced as a result of modification of the slr1841 promoter in the genome DNA, while the secretory production of useful substances can be facilitated as a result of deprivation of the outer membrane, resistance to antibiotics added from the outside of cells becomes degraded.

### Industrial Applicability

According to the present disclosure, it becomes possible to flexibly control the activity of a modified promoter. This makes it possible to readily select a promoter having an activity suitable for the type of the microorganism and the intended substance. Accordingly, the present disclosure can be applied to a wide variety of fields, such as food, chemical, pharmaceutical, and environmental fields.

## Claims

1. A modified promoter comprising:
a base sequence (SEQ ID NO: 1) located upstream of a start codon of a slr1841 gene, the base sequence consisting of 348 bases, or a base sequence having 80% or more homology with the base sequence represented by SEQ ID NO: 1, the base sequence being modified by at least one selected from the group consisting of (i) replacement of one or more bases included in the base sequence with another base, (ii) deletion of one or more bases included in the base sequence, and (iii) insertion of one or more bases into the base sequence.

2. The modified promoter according to claim 1, wherein:
in the base sequence represented by SEQ ID NO: 1, a region of a base sequence (SEQ ID NO: 2) extending from 54 to 94 bases upstream of the start codon, or a region corresponding to the region of the base sequence represented by SEQ ID NO: 2, is modified by at least one selected from the group consisting of (i) replacement of one or more bases included in the region with another base, (ii) deletion of one or more bases included in the region, and (iii) insertion of one or more bases into the region.

3. The modified promoter according to claim 1, wherein:
in the base sequence represented by SEQ ID NO: 1, a region of a base sequence TACAAT extending from 62 to 67 bases upstream of the start codon, or a region corresponding to the region of the base sequence TACAAT, is modified by at least one selected from the group consisting of (i) replacement of one or more bases included in the region with another base, (ii) deletion of one or more bases included in the region, and (iii) insertion of one or more bases into the region.

4. The modified promoter according to claim 1, wherein:
in the base sequence represented by SEQ ID NO: 1, a region of a base sequence TTCTCC extending from 89 to 94 bases upstream of the start codon, or a region corresponding to the region of the base sequence TTCTCC , is modified by at least one selected from the group consisting of (i) replacement of one or more bases included in the region with another base, (ii) deletion of one or more bases included in the region, and (iii) insertion of one or more bases into the region.

5. An expression vector comprising:
the modified promoter according to any one of claims 1 to 4.

6. A microorganism comprising:
the expression vector according to claim 5.

7. A microorganism comprising:
the modified promoter according to any one of claims 1 to 4 introduced in a genome.

8. A modified cyanobacterium comprising:
the modified promoter according to any one of claims 1 to 4 introduced in a genome.

9. A method for producing a substance, the method comprising:
cultivating the microorganism according to claim 6 to cause the microorganism to produce a substance.

10. A method for producing a substance, the method comprising:
cultivating the microorganism according to claim 7 to cause the microorganism to produce a substance.

11. A method for producing a substance, the method comprising:
cultivating the modified cyanobacterium according to claim 8 to cause the modified cyanobacterium to produce a substance.

12. A method for preparing a modified promoter, the method comprising:
modifying a base sequence (SEQ ID NO: 1) upstream of a start codon of a slr1841 gene, the base sequence consisting of 348 bases, or a base sequence having 80% or more homology with the base sequence represented by SEQ ID NO: 1, by at least one selected from the group consisting of (i) replacement of one or more bases included in the base sequence with another base, (ii) deletion of one or more bases included in the base sequence, and (iii) insertion of one or more bases into the base sequence.
